# FASCICULE DE BREVET EUROPEEN

(11) **EP 2 459 089 B1**
(45) Date de publication et mention de la délivrance du brevet: **07.09.2016**
(21) Numéro de dépôt: 10763204.4
(22) Date de dépôt: 29.07.2010
(51) Int. Cl.: A61B 17/70

(54) **DISPOSITIF EXTRA-DISCAL DE STABILISATION INTERVERTEBRAL**
ZUSATZSCHEIBEN-WIRBELKÖRPERSTABILISIERUNGSVORRICHTUNG
EXTRA-DISCAL INTERVERTEBRAL STABILIZATION DEVICE

(30) Priorité: 31.07.2009 FR 0955374
(43) Date de publication de la demande: 06.06.2012
(73) Titulaire: Spineway, 69500 Bron (FR)
(72) Inventeur: LE ROUX, Stéphane, F-69002 Lyon (FR); LAURITO, Philippe, F-83143 Le Val de France (FR)
(74) Mandataire: Cabinet Laurent & Charras
(86) Numéro de dépôt international: PCT/FR2010/051626
(87) Numéro de publication internationale: WO 2011/012829

(56) Documents cités:
- WO-A1-2007/124249
- FR-A1- 2 694 182
- US-A- 5 520 688
- US-A1- 2004 111 091
- US-A1- 2009 163 954
- US-A1- 2009 171 395

## Description

L'invention se rattache au secteur technique des ensembles de stabilisation intervertébrale.

D'une manière connue, le but recherché est de relier au moins deux vertèbres adjacentes, afin d'assurer une stabilisation rachidienne permettant une amplitude de mouvement entre les vertèbres considérées.

Différentes solutions techniques ont été proposées pour atteindre cet objectif. Par exemple, on connaît un système composé d'un ligament engagé dans un tube rigide disposé entre deux vis pédiculaires et relié auxdites vis. Ce tube rigide est positionné en compression entre les deux vis interdisant, par conséquent, tout mouvement entre les vis et le tube. Il en résulte un montage rigide générant des contraintes de charge, sollicitant les implants avec des risques de rupture des vis et des risques de descellement.

D'autres systèmes avec tige permettent un rapprochement des vertèbres, sans permettre toutefois un mouvement d'angulation entre lesdites vertèbres qui se déplacent en restant parallèles. Le mouvement en résultant n'est donc pas physiologique.

Selon l'enseignement du brevet FR 2.672.202, on connaît un implant chirurgical composé de vis pédiculaires et dont la tête présente au moins un épaulement radial délimitant au moins une zone périphérique de retenue d'un ligament. La liaison obtenue n'est pas suffisamment rigide et ne permet pas de limiter les mouvements, notamment en extension. Il en résulte une hyper sollicitation des articulations interfacétaires et une diminution de l'espace intervertébral permettant le passage des racines nerveuses. On observe également une hyper sollicitation du disque.

Une autre solution ressort de l'enseignement du brevet FR 2.694.182 qui décrit une attache pour prothèses inter-pédiculaires, comprenant une tige de fixation présentant une portée cylindrique dont l'extrémité est associée à une tête sphérique. Un ligament, dont les extrémités sont agencées en forme de boucles, relie les têtes sphériques de deux tiges consécutives. Le ligament peut, avant sa mise en place au niveau des têtes sphériques, traverser une bague de serrage. Cette solution n'est pas satisfaisante et demande un montage complexe nécessitant la présence d'un anneau monté sur la tête sphérique laquelle est montée avec capacité de déplacement sur la portée cylindrique de la tige de fixation. La bague n'est pas ajustée par rapport à la tête sphérique. Il en résulte une grande amplitude de mouvement.

L'invention s'est fixée pour but de remédier à ces inconvénients d'une manière simple, sûre, efficace et rationnelle.

Le problème que se propose de résoudre l'invention est d'obtenir une stabilisation dans le cadre de la thérapeutique des pathologies du rachis lombaire avec, pour objectif, de préserver la hauteur du foramen, la cohésion des facettes articulaires postérieures et à obtenir des micromouvements entre les deux vertèbres instrumentées.

Pour résoudre un tel problème, il a été conçu et mis au point un dispositif extra-discal de stabilisation intervertébrale comprenant au moins deux vis pédiculaires coopérant avec au moins deux vertèbres différentes, lesdites vis étant reliées par un ensemble de liaison comprenant au moins une partie rigide accouplée, à chaque extrémité, aux vis au moyen d'un élément.

Selon l'invention, la partie rigide est constituée par un corps cylindrique dont chacune des extrémités est rendue solidaire de l'élément qui est souple et déformable pour être lié à une partie de la vis pédiculaire correspondante de manière à créer un jeu réduit déterminé pour obtenir une amplitude limitée de mouvements multidirectionnels dans les plans sagittal, horizontal et frontal, tout en limitant un mouvement de translation selon l'axe de la vis.

Par jeu réduit, au sens de la présente demande, on entend la restauration de l'amplitude physiologique des mouvements entre les vertèbres.

Le dispositif peut être associé à d'autres systèmes d'implants (antérieurs, postérieurs, rigides ou non rigides) pour des indications d'arthrodèses ou de non arthrodèses. Ou bien, le dispositif peut constituer un système d'implant et il se suffit à lui-même ou il peut être combiné à un système postérieur rigide.

Dans le cas de l'association avec un système postérieur rigide, la stabilisation dynamique a pour but de diminuer le risque de dégénérescence du disque adjacent aux étages opérés.

Le but recherché est de permettre des micromouvements aux vertèbres, afin que la colonne se stabilise dans une position la plus anatomique possible.

Avantageusement, l'élément souple et réformable est un ligament.

Dans une forme de réalisation, l'élément, au niveau de sa liaison avec une partie de la vis pédiculaire, est conformé en boucle.

A partir de cette conception de base, plusieurs formes de réalisation peuvent être envisagées.

La partie en forme de boucle de l'élément souple est positionnée dans une gorge que présente la tête de vis pédiculaire correspondante.

La partie en forme de boucle de l'élément souple est positionnée dans une gorge que présente un embout apte à être fixé sur la vis pédiculaire correspondante.

Pour résoudre le problème posé de réduire le jeu entre la vis et le ligament, la partie rigide est constituée par un corps de section cylindrique pouvant être plate, méplate ou autre.

Dans une forme de réalisation, le corps est monobloc et creux au niveau de ses extrémités, l'élément souple étant engagé et fixé dans lesdites extrémités, de sorte que les boucles en débordent.

Dans une autre forme de réalisation, le corps cylindrique est réalisé en plusieurs éléments présentant des agencements d'accouplement.

Par exemple, le corps cylindrique est réalisé en trois éléments constitués par deux embouts d'extrémité rendus solidaires de la partie boucle de l'élément souple et d'un élément intermédiaire de liaison. L'élément intermédiaire de liaison présente, à chaque extrémité, des agencements d'accouplement coopérant avec des agencements complémentaires que présente chacun des embouts.

Selon une autre caractéristique, le corps présente un système dynamique permettant des mouvements de compression / distraction.

L'invention est exposée ci-après plus en détail à l'aide des figures des dessins annexés dans lesquels :
- Les figures 1, 2 et 3 montrent, par des vues en perspective, différentes formes de réalisation des têtes de vis pédiculaires pour l'accouplement de l'élément souple sous forme d'un ligament.
- Les figures 4 et 5 sont des vues en perspective montrant deux exemples de réalisation de l'élément souple associé à une tige rigide sous forme d'un corps cylindrique.
- Les figures 6 et 7 montrent par des vues en perspective une autre forme de réalisation selon laquelle l'élément de liaison est monté en combinaison avec des embouts venant se visser sur une portée filetée formée à l'extrémité des vis pédiculaires.
- Les figures 8, 9 et 10 montrent, par des vues partielles en perspective, différentes solutions pour l'accouplement de l'élément souple de section ronde ou de section méplate par rapport à la tête de vis.
- Les figures 11, 12, 13 et 14 montrent, par des vues en perspectives, des formes de réalisation selon lesquelles le corps cylindrique est réalisé en trois éléments présentant des agencements d'accouplement démontables.
- La figure 15 montre une forme de réalisation selon laquelle le corps présente un système de verrouillage avec contre-écrou.
- Les figures 16, 17 et 18 montrent, par des vues en perspectives, différents types de montage possibles sur un ou plusieurs étages.

Le dispositif extra-discal de stabilisation intervertébrale comprend au moins deux vis pédiculaires (V) coopérant avec au moins deux vertèbres différentes. Les vis (V) sont reliées par un ensemble de liaison composé d'un élément souple (1), notamment sous forme d'un ligament, associé à une partie rigide (2). La partie rigide (2) est accouplée, à chaque extrémité, aux têtes de vis (V) au moyen de l'élément souple (1) formant, à chaque extrémité, une boucle (1a). Le jeu entre la tête de vis considérée et le ligament (1), est déterminé pour créer un jeu réduit apte à permettre une amplitude limitée de mouvements dans les plans sagittal, horizontal et frontal.

Les mouvements autorisés se font autour de l'axe de la tête de vis pédiculaire (3). Le ligament (1) évite aux contraintes de se propager dans les vis et dans la partie rigide (2). Il en résulte une meilleure répartition des efforts entre l'ensemble de liaison et des corps vertébraux de l'étage opéré. La concentration de contraintes au niveau des implants étant évitée, les risques de ruptures sont réduits.

A partir de cette conception de base, différentes formes de réalisation peuvent être envisagées.

Le ligament (1), au niveau de la ou des parties agencées en forme de boucle (1a), est monté et positionné autour de la tête de vis (3) de la vis pédiculaire considérée (V).

Différentes solutions peuvent être envisagées au niveau de la réalisation de la tête de vis (3).

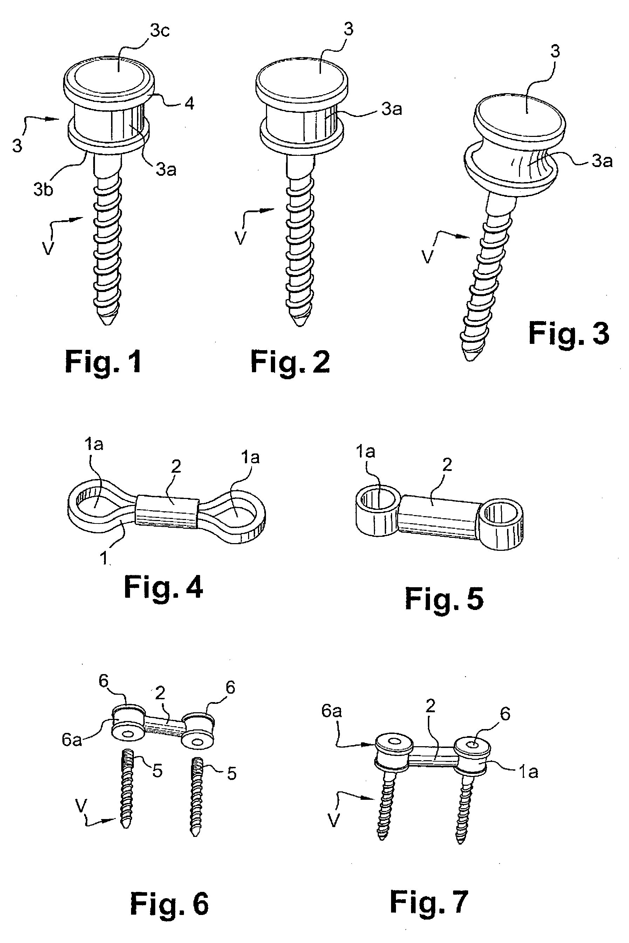

A la figure 1, la tête de vis (3) présente une portée cylindrique d'appui (3a) présentant, à sa base, une collerette de retenue (3b). L'extrémité supérieure de la portée cylindrique d'appui (3a) présente une portée filetée (3c) pour le montage d'un écrou (4).

A la figure 2, la portée cylindrique d'appui (3a), pour l'engagement de la boucle (1a) du ligament (1), est formée, directement lors de la fabrication de la tête de vis (3), ladite portée (3a) délimitant une gorge circulaire. Dans cet exemple de réalisation, le ligament (1) est de section méplate.

A la figure 3, la gorge circulaire (3a) présente une section sensiblement en forme de diabolo. Dans cette forme de réalisation, le ligament (1) est une section transversale ronde.

Dans une autre forme de réalisations, figures 6 et 7, les vis pédiculaires (V), présentent une tête (5) sous forme d'une portée filetée apte à recevoir, par vissage, des embouts (6) susceptibles d'être réalisés de la même façon que les têtes de vis (3), de manière à former une gorge (6a) pour le positionnement du ligament (1).

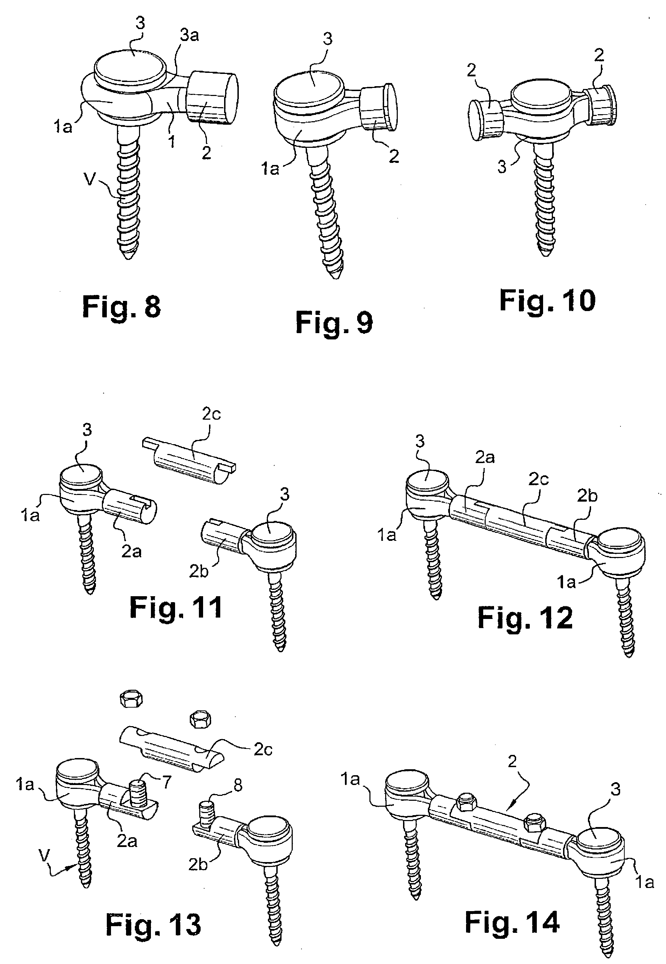

Les figures 8 et 9, notamment, montrent deux exemples de montage et de positionnement de la boucle (1a) d'un ligament de section ronde (figure 8) ou de section méplate (figure 9) par rapport à la tête de vis (3) ou à l'embout (6), quelle que soit leur forme de réalisation.

La boucle (1a) du ligament (1) peut, par exemple, être sertie en usine, à l'extrémité de la partie rigide (2), de manière à obtenir, entre la vis (V) et l'extrémité de l'élément rigide (2) où apparaît la boucle (1a) du ligament (1), un jeu (J) réduit et déterminé pour autoriser l'amplitude de mouvement, de sorte que les micromouvements autorisés se font, comme indiqué, autour de l'axe de la tête de vis pédiculaire, dans le sens sagittal, dans le plan horizontal et dans le plan frontal.

Sans pour cela sortir du cadre de l'invention, la liaison entre le ligament (1) et la tête de vis (3) peut s'effectuer par d'autres agencements qu'une boucle.

L'élément rigide (2) est réalisé, par exemple, sous forme d'un corps cylindrique, selon différents modes d'exécution. Par exemple, comme le montre la figure 4, le corps cylindrique (2) est monobloc et creux à chacune de ses extrémités, le ligament (1) étant engagé et fixé dans lesdites extrémités, de sorte que les boucles (1a) en débordent.

Aux figures 11 à 14, le corps cylindrique (2) est réalisé en plusieurs éléments présentant des agencements d'accouplement. Par exemple, le corps (2) est réalisé en trois éléments constitués par deux embouts d'extrémité (2a) et (2b) rendus solidaires, par tout moyen approprié, de la partie boucle (1a) du ligament (1). Les extrémités des deux embouts (2a) et (2b) présentent des agencements d'accouplement coopérant avec des agencements d'accouplement complémentaires d'un élément intermédiaire (2c). Ces agencements d'accouplement complémentaires peuvent être constitués, par exemple, par des agencements du type tenon-mortaise (figures 11 et 12) ou bien sous forme de pions (7) - (8) que présentent les embouts (2a) et (2b) et engagés dans des trous de l'élément intermédiaire (2c) pour coopérer avec des organes d'assemblages.

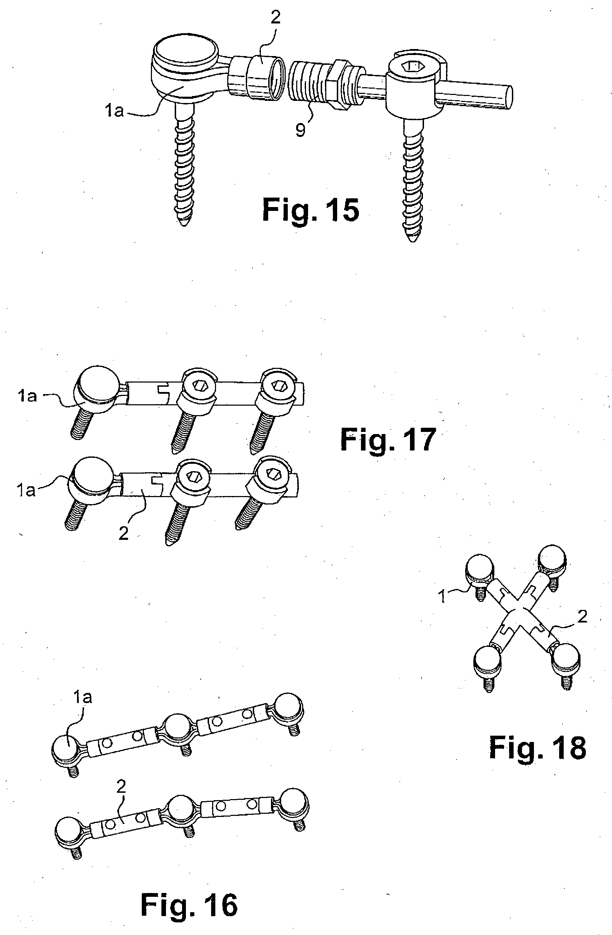

Dans une forme de réalisation illustrée à la figure 15, le corps (2) présente un système (9) de verrouillage avec contre-écrou permettant des mouvements de compression / distraction. Le corps (2) peut être monté avec un système dynamique.

Enfin, on renvoie aux figures 16, 17 et 18 qui montrent différents types de montage avec le dispositif de stabilisation dynamique selon l'invention.

La figure 16 montre un montage rigide sur deux étages.

La figure 17 montre un montage rigide sur un étage et souple sur un autre étage.

La figure 18 montre un montage en forme de croisillon.

Les avantages ressortent bien de la description.

## Revendications

1. Dispositif extra-discal de stabilisation intervertébral comprenant au moins deux vis pédiculaires (V) coopérant avec au moins deux vertèbres différentes, lesdites vis étant reliées par un ensemble de liaison, comprenant au moins une partie rigide (2) accouplée aux vis (V) au moyen d'un élément (1), **caractérisé en ce que** la partie rigide (2) est constituée par un corps cylindrique dont chacune des extrémités est rendue solidaire de l'élément (1), qui est souple et déformable, et formant à chacune desdites extrémités une boucle (1a), pour être lié à une partie de la vis pédiculaire correspondante de manière à créer un jeu réduit déterminé pour obtenir une amplitude limitée de mouvements multidirectionnels dans les plans sagittal, horizontal et frontal, tout en limitant un mouvement de translation selon l'axe de la vis.

2. Dispositif selon la revendication 1, **caractérisé en ce que** l'élément (1) souple et déformable est un ligament.

3. Dispositif selon l'une quelconque des revendications 1 et 2, **caractérisé en ce que** la boucle (1a) est positionnée dans une gorge (3a) que présente la tête de vis (3) pédiculaire correspondante (V).

4. Dispositif selon l'une quelconque des revendications 1 à 3, **caractérisé en ce que** la boucle (1a) est positionnée dans une gorge (6a) que présente un embout (6) apte à être fixé sur la vis pédiculaire correspondante.

5. Dispositif selon l'une quelconque des revendications 1 à 4, **caractérisé en ce que** le corps (2) est monobloc et creux à ses extrémités, l'élément (1) étant engagé et fixé dans lesdites extrémités du corps de sorte que les boucles (1a) en débordent.

6. Dispositif selon l'une quelconque des revendications 1 à 4, **caractérisé en ce que** le corps (2) est réalisé en plusieurs éléments présentant des agencements d'accouplement.

7. Dispositif selon la revendication 6, **caractérisé en ce que** le corps (2) est réalisé en trois éléments constitués par deux embouts d'extrémité (2a) et (2b) rendus solidaires de la partie boucle (1a) de l'élément (1) et d'un élément intermédiaire de liaison (2c).

8. Dispositif selon la revendication 7, **caractérisé en ce que** l'élément intermédiaire de liaison (2c) présente, à chaque extrémité, des agencements d'accouplement coopérant avec des agencements complémentaires que présente chacun des embouts.

9. Dispositif selon l'une quelconque des revendications 1 à 8, **caractérisé en ce que** le corps (2) présente un système permettant des mouvements de compression / distraction.

## Patentansprüche

1. Extradiskale Vorrichtung zur Zwischenwirbelstabilisierung mit mindestens zwei Pedikelschrauben (V), die mit mindestens zwei verschiedenen Wirbeln zusammenwirken, wobei die Schrauben durch eine Verbindungsvorrichtung miteinander verbunden werden, die mindestens einen starren Teil (2) umfasst, der mit den Schrauben (V) über ein Element (1) verbunden wird, **dadurch gekennzeichnet, dass** der starre Teil (2) aus einem zylinderförmigen Körper besteht, dessen Enden mit dem elastischen und verformbaren Element (1) fest verbunden sind, und welches an jedem Ende eine Schlinge (1a) bildet, um mit einem Teil der jeweiligen Pedikelschraube verbunden zu werden, so dass zur Erzielung einer begrenzten multidirektionalen Bewegungsamplitude in Sagittal-, Horizontal- und Frontalebene ein bestimmtes minimales Spiel geschaffen und zugleich die Translationsbewegung um die Schraubenachse begrenzt werden.

2. Vorrichtung nach Anspruch 1, **dadurch gekennzeichnet, dass** es sich bei dem elastischen und verformbaren Element (1) um ein Ligament handelt.

3. Vorrichtung nach einem der Ansprüche 1 und 2, **dadurch gekennzeichnet, dass** die Schlinge (1a) in eine an dem jeweiligen Pedikel-Schraubenkopf (V) ausgebildete Nut (3a) gelegt wird.

4. Vorrichtung nach einem der Ansprüche 1 bis 3, **dadurch gekennzeichnet, dass** die Schlinge (1a) in eine Nut (6a) eines Endstücks (6) gelegt wird, das an der jeweiligen Pedikelschraube befestigt werden kann.

5. Vorrichtung nach einem der Ansprüche 1 bis 4, **dadurch gekennzeichnet, dass** der Körper (2) aus einem Stück und an seinen Enden hohl ausgebildet ist, wobei das Element (1) derart in diese Körperenden eingefügt und dort befestigt wird, dass die Schlingen (1a) aus ihnen herausragen.

6. Vorrichtung nach einem der Ansprüche 1 bis 4, **dadurch gekennzeichnet, dass** der Körper (2) aus mehreren Elementen mit Verbindungsvorkehrungen besteht.

7. Vorrichtung nach Anspruch 6, **dadurch gekennzeichnet, dass** der Körper (2) aus drei Elementen besteht, welche aus zwei, mit dem Schlingenteil (1a) des Elements (1) fest verbunden Endstücken (2a) und (2b) und einem verbindenden Zwischenelement (2c) gebildet werden.

8. Vorrichtung nach Anspruch 7, **dadurch gekennzeichnet, dass** das verbindende Zwischenelement (2c) an jedem Ende Verbindungsvorkehrungen aufweist, die mit den am jeden Endstück befindlichen weiteren Vorkehrungen zusammenwirken.

9. Vorrichtung nach einem der Ansprüche 1 bis 8, **dadurch gekennzeichnet, dass** der Körper (2) aus eine System besteht, das Kompressions- und Distraktionsbewegungen ermöglicht.

## Claims

1. Extra-discal device for intervertebral stabilization including at least two pedicle screws (V) cooperating with at least two different vertebrae, the said screws being linked by a linkage assembly including at least one rigid part (2) coupled to the screws (V) by an element (1), **characterised in that** the rigid part (2) is composed of a cylindrical body, each end of which is fixed to the element (1) which is flexible and deformable, and forming at each of said ends a loop (1a), to be linked to a part of the corresponding pedicle screw so as to create reduced play so determined to obtain a limited range of multidirectional movements in the sagittal, horizontal and frontal planes, while limiting translational movement to the axis of the screw.

2. Device according to claim 1, **characterized in that** the flexible and deformable element (1) is a ligament.

3. Device according to any of claims 1 and 2, **characterized in that** the loop (1a) is positioned in a groove (3a) on the head (3) of the corresponding pedicle screw (V).

4. Device according to any of claims 1 to 3, **characterized in that** the loop (1a) is positioned in a groove (6a) on a head-piece (6) which can be fixed onto the corresponding pedicle screw.

5. Device according to any of claims 1 to 4, **characterized in that** the body (2) is monobloc and hollow at its ends, the element (1) being inserted and fixed in the said ends of the body in such a way that the loops (1a) protrude.

6. Device according to any of claims 1 to 4, **characterized in that** the body (2) is made in several parts with coupling arrangements.

7. Device according to claim 6, **characterized in that** the body (2) is made in three parts composed of two end-pieces (2a) and (2b) joined to the loop (1a) of the element (1) and an intermediate linking part (2c).

8. Device according to claim 7, **characterized in that** the intermediate linking part (2c) has coupling arrangements at each end, cooperating with complementary arrangements on each end-piece.

9. Device according to any of claims 1 to 8, **characterized in that** the body (2) has a system allowing compression/distraction movements.
